# EUROPÄISCHE PATENTSCHRIFT

(11) **EP 0 638 364 B1**
(45) Veröffentlichungstag und Bekanntmachung des Hinweises auf die Patenterteilung: **16.06.1999**
(21) Anmeldenummer: 94107894.1
(22) Anmeldetag: 21.05.1994
(51) Int. Cl.: B01L 3/00, G01N 33/80

(54) **Testbesteck**
Test device
Trousse d'essai

(30) Priorität: 03.08.1993 DE 9311574 U; 25.03.1994 DE 4410293
(43) Veröffentlichungstag der Anmeldung: 15.02.1995
(73) Patentinhaber: Baumgärtner, Erich Dr., D-69181 Leimen-Gauangelloch (DE)
(72) Erfinder: Baumgärtner, Erich Dr., D-69181 Leimen-Gauangelloch (DE)
(74) Vertreter: Meyer-Roedern, Giso, Dr.

(56) Entgegenhaltungen:
- EP-A- 0 051 748
- DE-A- 3 217 625
- DE-U- 9 012 557
- US-A- 3 033 412
- US-A- 4 472 357
- US-A- 4 829 006

## Beschreibung

Die Erfindung betrifft ein Testbesteck insbesondere zur Anwendung in der Hämatologie.

Zur Durchführung der nach den Richtlinien zur Blutgruppenbestimmung und Transfusion erforderlichen AB0-Identitätskontrolle (Bedside-Test) werden nach dem Stand der Technik verschiedene Halbfertig- oder Fertig-Testbestecke verwendet. So gibt es offene Testbestecke aus Papier, auf die Blut und Antiserum aufgetropft und zur Reaktion gebracht werden. Andere Systeme verwenden lyophilisierte bzw. angetrocknete Antiseren. Desweiteren bekannt sind Plastikkarten mit gebrauchsfertigen Flüssigseren in abgeschlossenen Kompartimenten (vgl. EP-A-0 051 748 und DE-U-90 12 557). Die Bedside-Testkarte gemäß DE-U-90 12 557 hat vier Kompartimente, von denen je zwei Serum Anti-A und Serum Anti-B enthalten. Mit dieser Karte kann z. B. eine AB0-Identitätskontrolle an einer Blutprobe des Patienten und einer Blutprobe aus einer Blutkonserve durchgeführt werden.

Die vorgegebene Zahl von vier Testfeldern (Kompartimenten) der Bedside-Testkarte ist nicht in jedem Fall optimal. So kann für homologe Anwendungen ein AB0-Test allein an einer Blutprobe des Patienten genügen. Dafür werden nur zwei der vier Testfelder benötigt. Werden außer dem Patientenblut auch die Konservenblute überprüft, was bei autologen Transfusionen vorgeschrieben ist, so benötigt man pro Konserve je zwei Testfelder zusätzlich.

Bei Testbestecken mit einer festen Zahl von Testfeldern bleiben oft entweder ungebrauchte Felder übrig, oder es stehen Testfelder nicht in der benötigten Zahl zur Verfügung. Dies führt zu Transfusionsverzögerungen und kompliziert die prätransfusionelle Diagnostik. Der Anfall an aufwendig zu entsorgendem Krankenhausmüll wird vergrößert, und es ergeben sich höhere Gesamtkosten.

Aus der US-A-4 472 357 ist ein Testbesteck mit einer Grundeinheit von wenigstens zwei zusammenhängenden Reagenzienträgern bekannt, die an ihrem Stoß eine Solltrennlinie aufweisen und sich durch Auftrennen der Solltrennlinie vereinzeln lassen.

Bei der US-A-4 829 006 sind Zentrifugierglasfläschchen an Sollbruchstellen miteinander verbunden.

Die DE-A-32 17 625 betrifft einen Reagenzglashalter mit Separiermöglichkeit an Solltrennlinien in Form von Perforationen. Der Reagenzglashalter kann mit ursprünglich einzelnen Reagenzgläsern bestückt werden.

Aus der US-A-3 033 412 ist eine Clipvorrichtung zur klammerartigen Verbindung je eines Paares Teströhrchen bekannt.

Aufgabe der Erfindung ist es, ein Testbesteck zu schaffen, das stets genau die benötigte Zahl von Testfeldern bereitstellt und bei einfacher, sicherer und schneller Handhabung eine vollständige Ausnutzung aller Testfelder gewährleistet.

Das diese Aufgabe lösende Testbesteck besteht aus einer Grundeinheit von wenigstens zwei zusammenhängenden Reagenzienträgern, die an ihrem Stoß eine Solltrennlinie aufweisen und sich durch Auftrennen der Solltrennlinie vereinzeln und im vereinzelten Zustand lösbar miteinander verbinden lassen in dem jeder Reagenzienträger wenigstens eine Rastnoppe und eine dazu passende Rastöffnung aufweist, die zusammenklipsbar sind.

Für die erwähnte AB0-Identitätskontrolle kommt ein Reagenzienträger mit zwei Kompartimenten (Testfeldern) für Serum Anti-A bzw. Anti-B in Betracht. Erfindungsgemäß werden mehrere solche Reagenzienträger zu einer Grundeinheit verbunden bereitgestellt. Die Zahl der Reagenzienträger pro Grundeinheit ist variabel und kann entsprechend den Bedürfnissen eines Krankenhauses, d. h. entsprechend der Anzahl der dort durchschnittlich pro Transfusionsvorgang verabreichten Blutkonserven, festgelegt werden. Dafür gibt es patienten- und behandlungsartspezifische Erfahrungswerte. Beispielsweise wird in einer Kinderklinik pro Transfusionsvorgang selten mehr als eine Blutkonserve verabreicht. Für eine Kinderklinik ist daher eine Grundeinheit mit zwei Reagenzienträgern für die AB0-Identitätskontrolle einer Blutprobe des Patienten und einer Blutprobe aus nur einer Blutkonserve angemessen. Weiter bespielsweise ist an einem Herz-Lungen-Operationszentrum mit einer Verabreichung von regelmäßig drei Blutkonserven pro Transfusionsvorgang zu rechnen. Hier empfiehlt sich eine aus vier Reagenzienträgern bestehende Grundeinheit.

Die als Grundeinheit in zusammenhängender Form bereitgestellten Reagenzienträger lassen sich an einer Solltrennlinie vereinzeln. Nicht benötigte Reagenzienträger können so abgetrennt und einer späteren Verwendung zugeführt werden. Dazu lassen sich die abgetrennten Reagenzienträger erfindungsgemäß auf lösbare Weise miteinander verbinden und so insbesondere wieder zu größeren Einheiten zusammenfassen, die Reagenzienträger in einer regelmäßig benötigten Zahl vereinen. Verzögerungen in der Behandlung von Patienten werden so vermieden, und es ist eine restlose Ausnutzung aller Reagenzienträger gewährleistet.

Durch die Erfindung wird ein frei kombinierbares System von abtrennbaren und wieder zusammenfügbaren Reagenzienträgern geschaffen. Jedem Krankenhaus werden patienten- und behandlungsartspezifische Grundeinheiten mit ein, zwei, drei oder mehr zusammenhängenden Reagenzienträgern zur Verfügung gestellt. Gegenüber der auch denkbaren Bereitstellung einzelner, nicht miteinander verbindbarer Reagenzienträger oder in jeweils unterschiedlicher Zahl fest und unlösbar miteinander verbundener Reagenzienträger führt das zu einer Vereinfachung in den organisatorischen Abläufen und der Lagerhaltung. Grundeinheiten mit einer sehr großen Zahl von Reagenzienträgern z. B. in Form von Band- oder Rollenware sind denkbar, mit Blick auf die vielfach erforderliche Lagerhaltung unter Kühlbedingungen, Stapelbarkeit u. a. für die meisten Anwendungen aber weniger vorteilhaft als Grundeinheiten mit zwei bis fünf Reagenzienträgern. Die Reagenzienträger lassen sich nach Bedarf beliebig vereinzeln und mit anderen vereinzelten Reagenzienträgern wieder zu Einheiten beliebiger Größe zusammenfügen, erneut lösen u.s.w. Unbenutzte Reagenzienträger müssen nie zwangsweise verworfen, sondern können einer späteren Verwendung zugeführt werden. Der Sondermüllanfall wird so erheblich reduziert, und es werden die Kosten für die Durchführung des Bedside-Tests infolge der Verwendbarkeit aller vorhandener Reagenzienträger minimiert. Das Lagerhaltungsproblem wird durch die Verwaltung standardisierter Grundeinheiten auf effektive Weise gelöst.

Hinzuweisen ist auf die Möglichkeit, durch Verbinden von Reagenzienträgern mit unterschiedlichen Nachweisreagenzien individuelle Tests zusammenzustellen und insbesondere Tests auf hämatologische und klinisch-chemische Parameter, z. B. Enzyme, Zucker o. ä. zu kombinieren.

Die Solltrennlinie zwischen den Reagenzienträgern kann eine Perforation oder ein unterbrochener Trennschnitt sein.

Bei einer herstellungstechnisch besonders vorteilhaften Variante bestehen die Reagenzienträger aus einem flachen Kunststoffstreifen, in den wenigstens ein Kompartiment zur Aufnahme eines Reagenz sowie gleichgerichtet damit zumindest eine Rastnoppe sind. Das kann durch Tiefziehen oder in einer anderen geeigneten Formtechnik geschehen.

Im Fall nur einer Rastnoppe und korrespondierenden Rastöffnung, die zum sicheren Verklipsen genügen, empfiehlt sich eine asymmetrische Anordnung, die die Unverwechselbarkeit sicherstellt. Die vereinzelten Reagenzienträger lassen sich in einer wohldefinierten ausgerichteten Stellung miteinander verklipsen. Die Anordnung der Reagenzienkompartimente, Testfelder o. ä. ist so auch eindeutig vorgegeben und unverwechselbar. Diese Unverwechselbarkeit ist sicherheitsrelevant für Patient und Arzt.

Die Rastöffnung kann eine Ausstanzung in dem Kunststoffstreifen sein.

Im Fall nur einer Rastöffnung und Rastnoppe empfiehlt es sich, diese in außermittiger, einander gegenüberliegender Anordnung am Rand des Kunststoffstreifens vorzusehen. Dadurch ist die Unverwechselbarkeit sichergestellt.

Rastöffnung und Rastnoppe sind vorzugsweise länglich, insbesondere länglich oval. Sie erstrecken sich vorzugsweise in Längsrichtung des Kunststoffstreifens. An die Rastnoppe können um ca. die Dicke des Kunststoffstreifens von dessen Unterseite versetzt beidseits ausladende Haltestege angeformt sein, die vorzugsweise von ovalen Wandausbuchtungen der Rastnoppe gebildet werden.

In einer anderen Variante hat die Rastnoppe die Form eines von dem Kunststoffstreifen abstehenden Stifts mit einer geringfügigen konischen Erweiterung nach außen und einer Spitze oder Fase am Ende.

Bei einer bevorzugten Variante ist auf die Reagenzienträger mit Überstand ein mehrlagiger Durchschreibe-Klebeetikettstreifen aufgebracht, der auf Höhe der Solltrennlinie am Stoß benachbarter Reagenzienträger eine Trennperforation aufweist. Der durchschreibende Selbstklebeetikettstreifen ermöglicht eine in den Richtlinien zur Bluttransfusion vorgeschriebene schriftliche Dokumentation des Testergebnisses sowohl an dem Testbesteck selbst, als auch in einem davon separaten Protokoll. Das beschriftete Etikett wird in das Protokoll eingeklebt, während der durchgeschriebene Etikettenträger an dem Testbesteck verbleibt. Die Trennperforation des Etikettenstreifens ermöglicht eine Vereinzelung von Etiketten zusammen mit den Reagenzienträgern, wenn deren Solltrennlinie aufgebrochen wird.

Der Reagenzienträger kann an einer Ecke eine Fase und der Klebeetikettstreifen hier Überstand haben. Das erleichtert das Abziehen des Etiketts.

Auch kann der Klebeetikettstreifen fahnenartig mit seitlichem Überstand an den Reagenzienträgern hängen. Bei breitem seitlichen Überstand des Selbstklebeetikettstreifens von den Reagenzienträgern kann in beträchtlichem Umfang Kunststoff-Trägermaterial eingespart werden. Es genügt, den Etikettstreifen an einer schmalen Randzone mit den Reagenzienträgern zu verkleben.

Bei einer bevorzugten Variante hat der Etikettstreifen eine sich über die Kleberandzone hinweg erstreckende Ausnehmung, die sich zu der Solltrennlinie hin öffnet und von der die Trennperforation abgeht. Das ermöglicht größere Toleranzen bei der Anbringung des Etikettstreifens an den Reagenzienträgern, da Solltrennlinie und Trennperforation nicht exakt auf gleicher Höhe liegen müssen, um ein einwandfreies Vereinzeln eines Reagenzienträgers mit daran hängendem Etikett zu gewährleisten.

Die Erfindung wird im folgenden anhand zweier in der Zeichnung dargestellter Ausführungsbeispiele einer Grundeinheit mit vier Reagenzienträgern näher erläutert. Es zeigen:
- Fig. 1: die Draufsicht auf ein erstes Testbesteck;
- Fig. 2: eine Seitenansicht des Testbestecks mit Blick in Richtung II von Fig. 1;
- Fig. 3: einen Schnitt durch das Testbesteck nach III - III von Fig. 1;
- Fig. 4: einen Schnitt durch das Testbesteck nach IV - IV von Fig. 1;
- Fig. 5: einen Schnitt durch das Testbesteck nach V - V von Fig. 1;
- Fig. 6: die Draufsicht auf ein zweites Testbesteck;
- Fig. 7: eine Seitenansicht des Testbestecks mit Blick in Richtung VII von Fig. 6; und
- Fig. 8: einen Schnitt durch das Testbesteck nach VIII - VIII von Fig. 7.

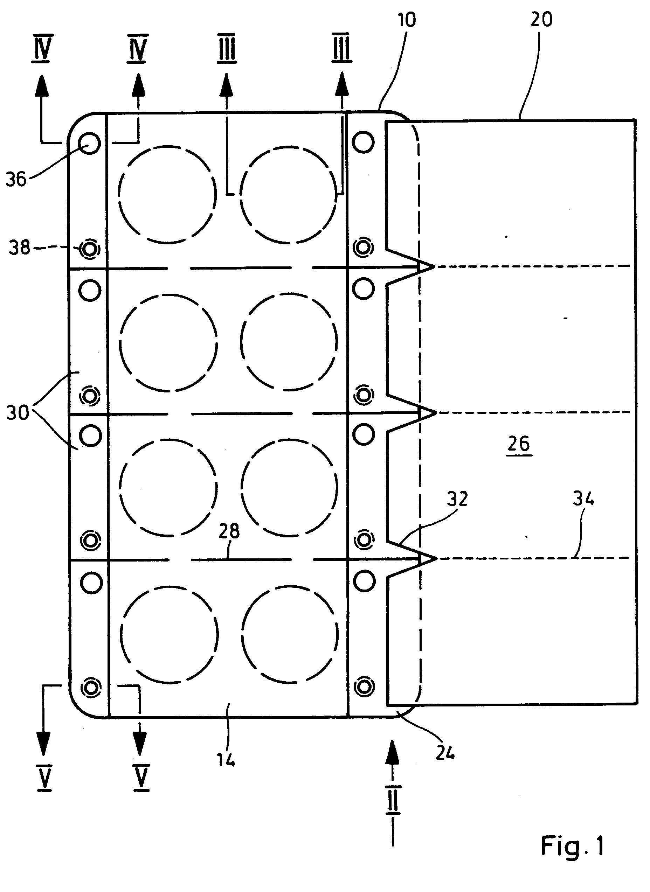

Das Testbesteck ist eine im wesentlichen rechteckige Karte 10 aus weißem Kunststoff. Die Karte 10 weist acht in einem rechteckigen Raster vier mal zwei angeordnete, durch Tiefziehen des Kartenmaterials erhaltene Kavitäten 12 auf. Die Kavitäten 12 haben eine kreisrunde, steilwandige Topfform. Sie dienen als Kompartiment für Seren Anti-A und Anti-B, mit denen eine ABO-Identitätskontrolle durchgeführt wird. Die in Fig. 1 linken Kavitäten 12 sind mit Serum Anti-A und die in Fig. 1 rechten Kavitäten 12 mit Serum Anti-B beschickt.

Die Kavitäten 12 sind mit einer durchsichtigen Kunststoffolie 14 überdeckt, die mit dem Kartenmaterial hermetisch verschweißt ist. Es handelt sich um eine elastische Doppelverbundfolie. Die Folie 14 schließt mit der Wand der Kavitäten 12 einen Kapillarspalt 16 ein, in den Serum vom Boden 18 der Kavitäten 12 aufsteigt. Die Seren Anti-A und Anti-B sind unterschiedlich eingefärbt. Die Seren in dem Kapillarspalt 16 bilden einen farbigen Halo an der Peripherie der Kavitäten 12, während der Boden 18 der Kavitäten 12 in der Hintergrundfarbe weiß des Kartenmaterials erscheint.

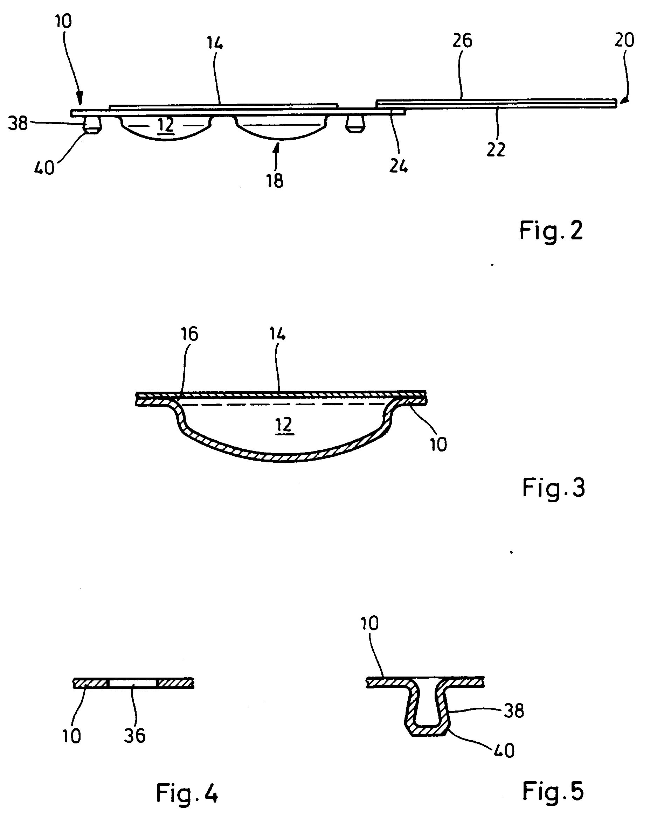

Die Karte 10 gemäß Fig. 1 bis Fig. 5 ist an einem ihrer Seitenränder mit einem fahnenartig zur Seite abstehenden Durchschreibe-Klebeetikettstreifen 20 versehen. Der Klebeetikettstreifen 20 ist mehrlagig. Er hat einen Träger 22 z. B. aus Silikonpapier, der an einer schmalen Randzone 24 der Karte 10 mit letzterer dauerhaft verklebt ist. Auf den Träger 22 ist mit einem Haftkleber ein Papieretikett 26 aufgebracht, das sich von dem Träger 22 abziehen läßt, um an anderer Stelle aufgeklebt zu werden. Das Etikett 26 kann beschriftet werden und schreibt dabei auf den Träger 22 durch.

Von der Karte 10 sind durch drei parallele, jeweils mehrmals unterbrochene Trennschnitte 28 vier Streifen abgeteilt, die je einen Reagenzienträger 30 mit einer Serum Anti-A und Serum Anti-B Kavität 12 bilden. Die Reagenzienträger 30 lassen sich durch Aufreißen der Trennschnitte 28 vereinzeln.

Der Klebeetikettstreifen 20 hat an dem seitlichen Rand, mit dem er auf die Karte 10 aufgeklebt ist, drei dreieckige Ausnehmungen 32, die tiefer sind als die Kleberandzone 24 der Karte 10. Die Ausnehmungen 32 kommen auf Höhe der Trennschnitte 28 in der Karte 10 zu liegen. Sie öffnen sich zu den Trennschnitten 28 hin. Von dem Scheitel der Ausnehmungen 32 gehen Trennperforationen 34 des Klebeetikettstreifens 20 ab, die sich parallel zu den Trennschnitten 28 über dessen volle Breite erstrecken. Wird ein Reagenzienträger 30 an einem der Trennschnitte 28 vereinzelt, so reißt die zugehörige Trennperforation 34 mit auf, und es bleibt ein vereinzeltes Etikett an dem Reagenzienträger 30 hängen.

An den vier Reagenzienträgern 30 der Karte 10 sind Rastmittel vorgesehen, die nach dem Prinzip von Nut und Feder zusammenwirken und ein Zusammenklipsen vereinzelter Reagenzienträger 30 zu größeren Einheiten ermöglichen. In Fig. 1 bis Fig. 5 erkennt man je zwei Rastöffnungen 36 und darin passende Rastnoppen 38 an den Reagenzienträgern 30, die in zu den Trennschnitten 28 parallelen Reihen angeordnet sind und einander gegenüberliegen. Die Rastöffnungen 36 sind aus dem Kartenmaterial ausgestanzte Rundlöcher. Die Rastnoppen 38 sind Rundstifte, die zugleich mit den Kavitäten 12 zu derselben Seite hin aus dem Kartenmaterial tiefgezogen sind. Die Rastnoppen 38 haben eine geringfügige konische Erweiterung nach außen und eine Spitze oder Fase 40 zum Einführen in die Rastöffnungen 36.

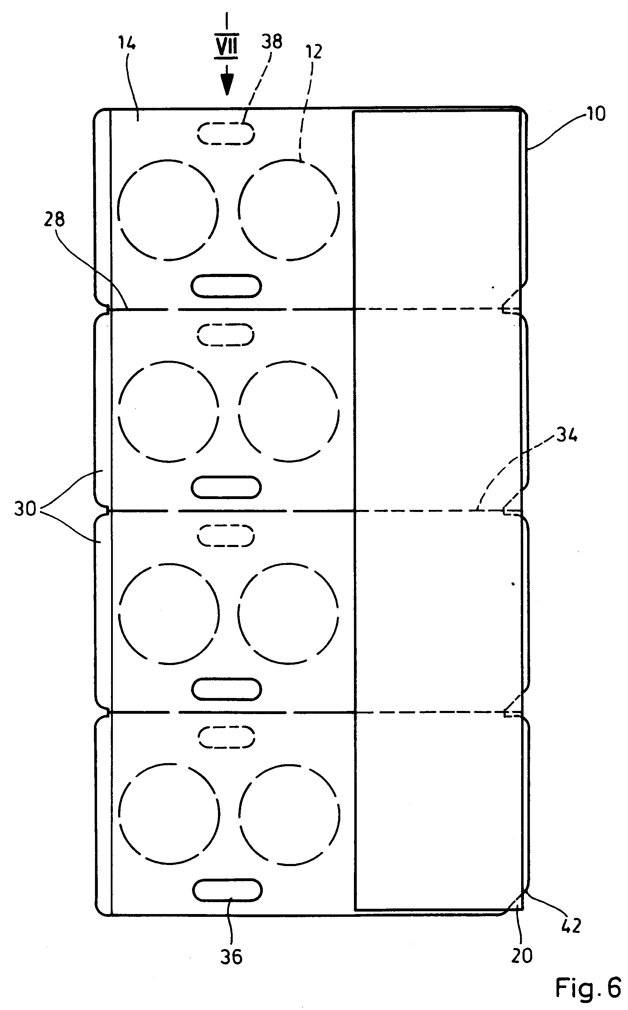

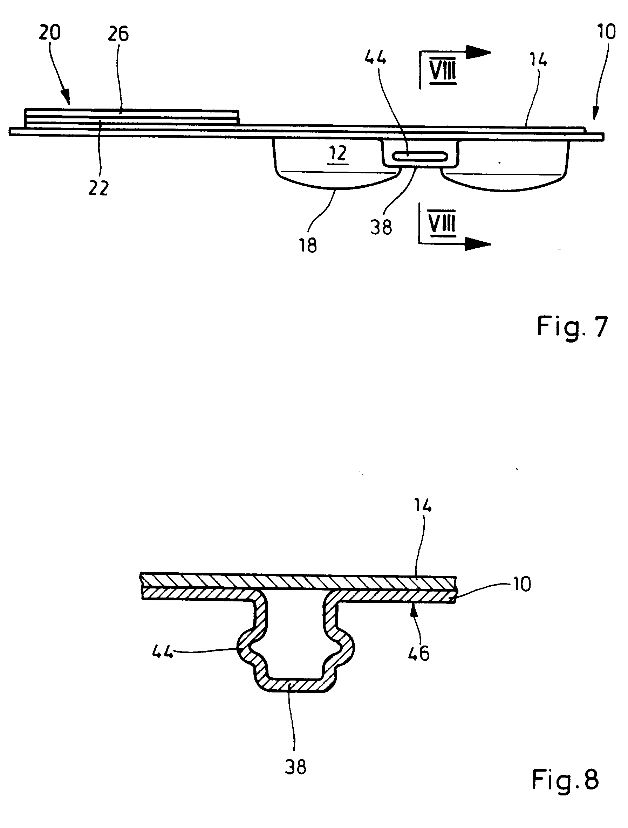

Bei dem Testbesteck gemäß Fig. 6 bis Fig. 8 ist die Karte 10 gänzlich mit der Kunststoffolie 14 überdeckt. Das Testbesteck hat einen Klebeetikettstreifen 20, der mit annähernd seiner vollen Fläche auf die Kunststoffolie 14 aufgeklebt ist. Eine Trennperforation 34 des Klebeetikettstreifens 20 kommt mit mehrfach unterbrochenen Trennschnitten 28 zur Deckung, die die Karte 10 und die Kunststoffolie 14 durchsetzen. Die Karte 10 hat an einer Ecke eine Fase 42. Der Klebeetikettstreifen 20 hat hier mit seinem Träger 22 und Papieretikett 26 Überstand von der Karte 10. Im Bereich des Überstands sind Träger 22 und Papieretikett 26 nicht miteinander verklebt. Dadurch wird das Abziehen des Papieretiketts 26 von dem Träger 22 erleichtert.

Die vier Reagenzienträger 30 der Karte 10 gemäß Fig. 6 bis Fig. 8 haben je eine Rastöffnung 36 und eine Rastnoppe 38, die ein Zusammenklipsen vereinzelter Reagenzienträger 30 ermöglichen. Die Rastöffnung 36 ist ein aus der Karte 10 und der sie überdeckenden Kunststoffolie 14 ausgestanztes ovales Langloch, das sich zwischen den Kavitäten 12 am Rand des Reagenzienträgers 30 befindet und sich in dessen Längsrichtung erstreckt. Die Rastnoppe 38 ist eine im Grundriß ovale Vertiefung der Karte 10, die zugleich mit den Kavitäten 12 zu derselben Seite hin aus dem Kartenmaterial tiefgezogen ist. Die Rastnoppe 38 befindet sich an dem der Rastöffnung 36 gegenüberliegenden Rand des Reagenzienträgers 30. Sie ist etwas kürzer als die Rastöffnung 36, erstreckt sich wie diese in Längsrichtung des Reagenzienträgers 30 und liegt der Rastöffnung 36 mit mittiger Ausrichtung gegenüber.

Die Rastnoppe 38 hat an ihren Längsseiten Wandausbuchtungen 44, die um etwas mehr als die Dicke eines Reagenzienträgers 30 von dessen Unterseite 46 versetzt sind, ihrerseits ovalen Grundriß haben und sich in Längsrichtung der Rastnoppe 38 erstrecken. Die Wandausbuchtungen 44 bilden Haltestege für die mit leichtem Spiel zusammengeklipsten Reagenzienträger.

Das beschriebene Testbesteck mit vier Reagenzienträgern 30 bildet eine Grundeinheit für den AB0-Bedside-Test bei regelmäßiger Transfusion von drei Blutkonserven. Ein Reagenzienträger 30 wird mit Patientenblutproben, und die drei anderen Reagenzienträger mit Blutproben aus je einer Blutkonserve beschickt, indem die Kunststoffolie 14 mit einer die Probe enthaltenden Kanüle, Spritze, Pipettenspitze o. ä., durchstochen und ein Tropfen Blut in die Kavität 12 gegeben wird. Die stattfindende Agglutinationsreaktion wird beobachtet, bewertet und protokolliert. Die Reagenzienträger 30 bleiben dabei im Kartenverbund, und auch der Durchschreibe-Klebeetikettstreifen 20 wird insgesamt von der Karte 10 abgezogen und in ein Protokoll eingeklebt.

Werden einmal nicht alle Reagenzienträger 30 für den Bedside-Test benötigt, so können unbenutzte Reagenzienträger 30 wie beschrieben vereinzelt und wieder zu größeren Einheiten zusammengeklipst werden.

### Liste der Bezugszeichen

- 10: Karte
- 12: Kavität
- 14: Kunststoffolie
- 16: Kapillarspalt
- 18: Boden
- 20: Durchschreibe-Klebeetikettstreifen
- 22: Träger
- 24: Randzone
- 26: Papieretikett
- 28: Trennschnitt
- 30: Reagenzienträger
- 32: Ausnehmung
- 34: Trennperforation
- 36: Rastöffnung
- 38: Rastnoppe
- 40: Fase
- 42: Fase
- 44: Wandausbuchtung
- 46: Unterseite

## Patentansprüche

1. Testbesteck mit einer Grundeinheit von wenigstens zwei zusammenhängenden Reagenzienträgern (30), die an ihrem Stoß eine Solltrennlinie aufweisen und durch Auftrennen der Solltrennlinie vereinzelbar sind, dadurch gekennzeichnet, daß die vereinzelten Reagenzienträger (30) durch Zusammenklipsen lösbar miteinander verbindbar sind, wozu an einem jeden Reagenzienträger (30) wenigstens eine Rastnoppe (38) und eine dazu passende Rastöffnung (36) vorgesehen ist.

2. Testbesteck nach Anspruch 1, dadurch gekennzeichnet, daß die Reagenzienträger (30) aus einem flachen Kunststoffstreifen bestehen, in den wenigstens ein Kompartiment zur Aufnahme eines Reagenz sowie gleichgerichtet damit die Rastnoppe (38) eingeformt ist.

3. Testbesteck nach Anspruch 1 oder 2, dadurch gekennzeichnet, daß die Rastöffnung (36) eine Ausstanzung in dem Kunststoffstreifen ist.

4. Testbesteck nach einem der Ansprüche 1 bis 3, dadurch gekennzeichnet, daß im Fall nur einer Rastöffnung (36) und Rastnoppe (38) diese in außermittiger, einander gegenüberliegender Anordnung am Rand des Kunststoffstreifens vorgesehen sind.

5. Testbesteck nach einem der Ansprüche 1 bis 4, dadurch gekennzeichnet, daß Rastöffnung (36) und Rastnoppe (38) länglich, insbesondere länglich oval sind und sich vorzugsweise in Längsrichtung des Kunststoffstreifens erstrecken.

6. Testbesteck nach einem der Ansprüche 1 bis 5, dadurch gekennzeichnet, daß um ca. die Dicke des Kunststoffstreifens von dessen Unterseite (46) versetzt beidseits ausladende Haltestege an die Rastnoppe (38) angeformt sind.

7. Testbesteck nach Anspruch 6, dadurch gekennzeichnet, daß die Haltestege länglich ovale Wandausbuchtungen (44) der Rastnoppe (38) sind.

8. Testbesteck nach einem der Ansprüche 1 bis 4, dadurch gekennzeichnet, daß die Rastnoppe (38) die Form eines von dem Kunststoffstreifen abstehenden Stifts mit einer geringfügigen konischen Erweiterung zu ihrem von dem Kunststoffstreifen abstehenden Ende und einer Spitze oder Fase (40) am Ende hat.

9. Testbesteck nach einem der Ansprüche 1 bis 8, dadurch gekennzeichnet, daß auf die Reagenzienträger (30) mit Überstand ein mehrlagiger Durchschreibe-Klebeetikettstreifen (20) aufgebracht ist, der auf Höhe der Solltrennlinie am Stoß benachbarter Reagenzienträger (30) eine Trennperforation (34) aufweist.

10. Testbesteck nach Anspruch 9, dadurch gekennzeichnet, daß der Reagenzienträger (30) an einer Ecke eine Fase (42) und der Klebeetikettstreifen (20) hier Überstand hat.

11. Testbesteck nach Anspruch 9 oder 10, dadurch gekennzeichnet, daß der Klebeetikettstreifen (20) fahnenartig mit seitlichem Überstand an den Reagenzienträgern (30) hängt und an einer schmalen Randzone (24) der Reagenzienträger (30) mit letzteren verklebt ist.

12. Testbesteck nach Anspruch 11, dadurch gekennzeichnet, daß der Klebeetikettstreifen (20) eine sich über die Randzone (24) hinweg erstreckende Ausnehmung (32) hat, die sich zu der Solltrennlinie hin öffnet und von der die Trennperforation (34) abgeht.

## Claims

1. A test apparatus with a basic unit of at least two contiguous reagent carriers (30) which have a nominal dividing line at their joint and which are separable by tearing along the nominal dividing line, characterised in that the separated reagent carriers (30) can be releasably connected to one another by being clipped together, for which purpose each reagent carrier (30) is provided with at least one locating projection (38) and a complementary locating aperture (36).

2. A test apparatus according to Claim 1, characterised in that the reagent carriers (30) consist of a flat plastics strip, in which is formed at least one compartment to accommodate a reagent and, in he same direction, the locating projection (38).

3. A test apparatus according to Claim 1 or 2, characterised in that the locating aperture (36) is punched in the plastics strip.

4. A test apparatus according to any one of Claims 1 to 3, characterised in that if there is only one locating aperture (36) and locating projection (38), they are provided in an off-centre, mutually opposite arrangement at the edge of the plastics strip.

5. A test apparatus according to any one of Claims 1 to 4, characterised in that the locating aperture (36) and the locating projection (38) are elongate, in particular elongate oval, and extend preferably in the longitudinal direction of the plastics strip.

6. A test apparatus according to any one of Claims 1 to 5, characterised in that retaining webs, are formed on the underside (46) of the plastics strip protruding by approximately the thickness thereof, positioned on either side of the locating projection (38).

7. A test apparatus according to Claim 6, characterised in that the retaining webs are elongate oval wall protrusions (44) of the locating projection (38).

8. A test apparatus according to any one of Claims 1 to 4, characterized in that the locating projection (38) is in the shape of a stud projecting from the plastics strip, having a slight conical widening towards its end remote from the plastics strip and having a tip or bevel (40) at its end.

9. A test apparatus according to any one of Claims 1 to 8, characterised in that a multilayer duplicating adhesive label strip (20) is applied to the reagent carriers (30) and projects thereover, which has a separable perforated line (34) at the level of the nominal dividing line at the joint of adjacent reagent carriers (30).

10. A test apparatus according to Claim 9, characterised in that the reagent carrier (30) has a bevel (42) at one corner and at this location the adhesive label strip (20) projects thereover.

11. A test apparatus according to Claim 9 or 10, characterised in that the adhesive label strip (20) is attached to the reagent carriers (30) in a tab-like manner so as to project laterally thereover and is adhered to the reagent carriers (30) in a narrow edge zone (24) thereof.

12. A test apparatus according to Claim 11, characterised in that the adhesive label strip (20) has a notch (32) which extends over the edge zone (24), which opens towards the nominal dividing line and from which the separable perforated line (34) extends.

## Revendications

1. Trousse d'essai avec une unité de base d'au moins deux supports à réactifs (30) contigus, qui présentent une ligne de séparation théorique sur leur jointure et peuvent être isolés en défaisant la ligne de séparation théorique, caractérisée en ce que les différents supports à réactifs (30) peuvent être reliés entre eux de façon amovible en les clipsant ensemble, opération pour laquelle il est prévu sur chaque support à réactif (30) au moins un bouton d'encliquetage (38) et un orifice d'encliquetage (36) adapté.

2. Trousse d'essai selon la revendication 1, caractérisée en ce que les supports à réactifs (30) sont à base d'une bande en matière synthétique plate, dans laquelle est formé au moins un compartiment pour loger un réactif et le bouton d'encliquetage (38), dirigé dans le même sens.

3. Trousse d'essai selon la revendication 1 ou 2, caractérisée en ce que l'orifice d'encliquetage (36) est une partie découpée dans la bande plastique.

4. Trousse d'essai selon l'une quelconque des revendications 1 à 3, caractérisée en ce que, dans le cas d'un seul orifice d'encliquetage (36) et d'un seul bouton d'encliquetage (38), ceux-ci sont disposés de façon excentrée, l'un en face de l'autre, au bord de la bande en matière synthétique.

5. Trousse selon l'une quelconque des revendications 1 à 4, caractérisée en ce que l'orifice d'encliquetage (36) et le bouton d'encliquetage (38) sont longitudinaux, en particulier ovales et s'étendent de préférence dans le sens longitudinal de la bande en matière synthétique.

6. Trousse d'essai selon l'une quelconque des revendications 1 à 5, caractérisée en ce que des barrettes d'arrêt en porte à faux des deux côtés et décalées d'environ l'épaisseur de la bande en matière synthétique par rapport à son côté inférieur (46) sont formées sur le bouton d'encliquetage (38).

7. Trousse d'essai selon la revendication 6, caractérisée en ce que les barrettes d'arrêt sont des courbures de paroi (44) ovales du bouton d'encliquetage (38).

8. Trousse d'arrêt selon l'une quelconque des revendications 1 à 4, caractérisée en ce que le bouton d'encliquetage (38) a la forme d'une goupille débordant de la bande en matière synthétique avec une extension conique légère vers son extrémité dépassant de la bande en matière synthétique et une pointe ou un chanfrein (40) à l'extrémité.

9. Trousse d'essai selon l'une quelconque des revendications 1 à 8, caractérisée en ce que sur les supports à réactifs (30) est appliquée avec un débordement une bande autocopiante à étiquette adhésive (20) à plusieurs épaisseurs, qui présente une perforation de séparation (34) au niveau de la ligne de séparation théorique à la jointure des supports de réactif (30) voisins.

10. Trousse d'essai selon la revendication 9, caractérisée en ce que le support à réactif (30) a un chanfrein (42) à un angle et la bande à étiquette adhésive (20) a ici un débordement.

11. Trousse d'essai selon la revendication 9 ou 10, caractérisée en ce que la bande à étiquette adhésive (20) est accrochée à la façon d'un drapeau avec un débordement latéral sur les supports à réactifs (30) et est collée sur une zone périphérique (24) étroite des supports à réactifs (30) avec celle-ci.

12. Trousse d'essai selon la revendication 11, caractérisée en ce que la bande à étiquette adhésive (20) a un évidement (32) qui s'étend au-delà de la zone périphérique (24), s'ouvre en direction de la ligne de séparation théorique et de laquelle part la perforation de séparation (34)
